# EUROPÄISCHE PATENTSCHRIFT

(11) **EP 3 924 111 B1**
(45) Veröffentlichungstag und Bekanntmachung des Hinweises auf die Patenterteilung: **03.09.2025**
(21) Anmeldenummer: 20705898.3
(22) Anmeldetag: 11.02.2020
(51) Int. Cl.: B08B 1/12, B08B 1/34, B08B 1/52, B01F 33/841, B01F 35/12, A61L 2/20, A61L 2/18

(54) **VERFAHREN ZUR VERMEIDUNG DES MIKROBIELLEN BEFALLS EINER REINIGUNGSVORRICHTUNG FÜR EINE DOSIERANLAGE UND REINIGUNGSVORRICHTUNG**
METHOD FOR AVOIDING THE MICROBIAL ATTACK OF A CLEANING APPARATUS FOR A METERING UNIT, AND CLEANING APPARATUS
PROCÉDÉ PERMETTANT D'ÉVITER UNE ATTAQUE MICROBIENNE D'UN DISPOSITIF DE NETTOYAGE DESTINÉ À UNE INSTALLATION DE DOSAGE ET DISPOSITIF DE NETTOYAGE

(30) Priorität: 11.02.2019 DE 102019103273
(43) Veröffentlichungstag der Anmeldung: 22.12.2021
(73) Patentinhaber: Brillux GmbH & Co. KG, 48163 Münster (DE)
(72) Erfinder: HÖRSTING, Ingo, 48317 Drensteinfurt (DE); ERBER, Dietmar, 48143 Münster (DE); STORB, Rainer, 48301 Nottuln (DE); FELS, Sven, 48153 Münster (DE)
(74) Vertreter: Cohausz & Florack
(86) Internationale Anmeldenummer: PCT/EP2020/053385
(87) Internationale Veröffentlichungsnummer: WO 2020/165118

(56) Entgegenhaltungen:
- EP-A1- 1 990 102
- DE-A1- 10 248 561
- DE-B1- 2 745 853
- JP-A- 2007 167 837
- JP-A- H04 107 824

## Beschreibung

Die Erfindung betrifft ein Verfahren zur Vermeidung des mikrobiellen Befalls einer Reinigungsvorrichtung für eine Dosieranlage, wobei die Reinigungsvorrichtung wenigstens ein mechanisches Reinigungselement mit wenigstens einer Reinigungsfläche aufweist. Ferner betrifft die Erfindung ein Verfahren zum Reinigen einer Dosieranlage, eine Reinigungsvorrichtung sowie eine Dosieranlage.

Dosieranlagen ermöglichen die präzise Bereitstellung einer bestimmten Menge einer Dispersion auf Abfrage. Im Sinne der vorliegenden Erfindung sind mit Dosieranlagen grundsätzlich sämtliche Systeme gemeint, die ein dosiertes Bereitstellen von einem Material ermöglichen. Dosieranlagen kommen insbesondere beim Abmischen von Dispersionsfarben zur Anwendung.

Eine Dosieranlage für Farben ist beispielsweise in der DE 196 54 829 A1 beschrieben. Als besonderes Problem bei Dosieranlagen für Dispersionen erweist sich deren mikrobieller Befall durch Pilze und Bakterien. Dies ist auf eine Lagerung der Dispersion(en) in einem entsprechenden Behälter in der Dosieranlage zum Teil über mehrere Wochen und Monate zurückzuführen. Der mikrobielle Befall ist dabei nicht auf den Lagerungsbehälter in der Dosieranlage beschränkt, sondern verteilt sich auf sämtliche Elemente der Dosieranlage, die mit der befallenen Dispersion in Kontakt kommen, insbesondere auch auf die Dosiereinheit, mittels derer die gelagerte Dispersion in kontrollierter Menge abgegeben werden kann. Die Dosiereinheit umfasst üblicherweise einen sogenannten Pumpenkopf, über welchen die Dispersion aus der Dosieranlage abgegeben werden kann. Mit der Zeit setzen sich der Pumpenkopf und insbesondere dessen Auslassöffnung bzw. -düse mit Dispersionsresten zu, so dass eine regelmäßige, in der Regel mechanisch durchgeführte Reinigung dieser Auslassöffnung notwendig ist. Hierzu sind aus der Praxis Reinigungsvorrichtungen bekannt, welche beispielsweise eine rotierende Bürste umfassen, mithilfe derer die Reinigung der Auslassöffnung zuverlässig erfolgt. Die rotierende Bürste ihrerseits wird im Betrieb der Reinigungsvorrichtung in einem Reinigungsbad gereinigt bzw. gespült, in welches sie im Reinigungsbetrieb teilweise eingetaucht ist. Wird das Reinigungsbad nicht regelmäßig gewechselt, so können sich Pilze und Bakterien bilden, welche die Reinigungsvorrichtung kontaminieren. Weist die Dosieranlage eine Mehrzahl von Lagerungsbehältern für Dispersionen mit dementsprechend einer Mehrzahl von Pumpenköpfen auf, wie dies beispielsweise bei Farbmischanlagen der Fall ist, so kann sich der mikrobielle Befall einer gelagerten Dispersion auch auf die anderen Dispersionen ausbreiten. Dies kann dadurch erfolgen, dass die befallenen Dispersionsrückstände an der Auslassöffnung oder -düse eines Pumpenkopfes abgereinigt werden und sich sodann in dem Reinigungsbad sammeln. Eine Verteilung des mikrobiellen Befalls auf die Pumpenköpfe der zunächst nicht befallenen Dispersionen erfolgt sodann über die Reinigungsbürste, die ihrerseits permanent im Reinigungsbad gespült wird.

Die JP H04 107824 A offenbart eine Reinigungsvorrichtung für Halbleiterwafer die eine Rollbürste umfasst, wobei die Rollbürste in einem Waschbehälter mit Ozonwasser gewaschen wird.

Die DE 102 48 561 A1 beschreibt eine Vorrichtung zum Melken und ein Verfahren zum Desinfizieren von melktechnischen Komponenten. Insbesondere eignet sich das Verfahren auch für den Einsatz zur Desinfektion von (rotierenden) Reinigungsbürsten zur Euterreinigung.

Die DE 27 45 853 B1 beschreibt eine Farbtonmischvorrichtung mit einem karusselartig ausgebildeten Vorratsregal mit mehreren mit je einem steuerbaren Auslaufventil versehenen Farbbehältern und einer Reinigungsvorrichtung.

Die EP 1 990 102 A1 beschreibt eine Abgabevorrichtung mit einer Reinigungsvorrichtung zum Reinigen einer Abgabeöffnung, wobei die Reinigungsvorrichtung eine Reinigungsbürste und ein Flüssigkeitsreservoir mit einer Reinigungsflüssigkeit umfasst.

Die JP 2007 167837 A beschreibt, nach dem Oberbegriff des Anspruchs 1, eine Reinigungsvorrichtung für eine Auslassdüse einer Beschichtungsvorrichtung, die eine Rotationswalze zur Aufnahme von aus der Auslassdüse austretendender Beschichtungsflüssigkeit umfasst.

Der vorliegenden Erfindung liegt daher die Aufgabe zugrunde, ein Verfahren anzugeben, mithilfe dessen der mikrobielle Befall einer Reinigungsvorrichtung für eine Dosieranlage wirksam vermieden wird.

Eine weitere Aufgabe der vorliegenden Erfindung besteht ferner darin, eine Reinigungsvorrichtung für eine Dosieranlage, insbesondere für eine Dispersion, speziell für eine Farbdosieranlage, anzugeben, bei der ein mikrobieller Befall der Reinigungsvorrichtung sowie der Dosieranlage wirksam vermieden wird.

Weitere Aufgaben ergeben sich aus den nachfolgenden Ausführungen.

Die vorstehenden Aufgaben werden verfahrensmäßig mit einem Verfahren gemäß dem Oberbegriff des Anspruches 1 dadurch gelöst, dass die wenigstens eine Reinigungsfläche des wenigstens einen mechanischen Reinigungselements zumindest zeitweise mit einem Oxidationsmittel beaufschlagt wird, wobei das Oxidationsmittel ein gasförmiges Oxidationsfluid ist und wobei das Oxidationsfluid Ozon enthält.

Der besondere Vorteil des erfindungsgemäßen Verfahrens besteht darin, dass durch Beaufschlagung des wenigstens einen mechanischen Reinigungselements mit einem Oxidationsmittel eine Anlagerung und eine folgende Vermehrung von mikrobiellem Befall, also beispielsweise von Bakterien, Schimmel oder Hefepilzen, wirksam über einen langen Zeitraum verhindert wird. Bei bereits eingesetztem mikrobiellem Befall erlaubt das erfindungsgemäße Verfahren, diesen sicher zu eliminieren.

Überraschend hat sich gezeigt, dass mit dem erfindungsgemäß Verfahren zur Vermeidung des mikrobiellen Befalls einer Reinigungsvorrichtung in einer Dosieranlage, speziell einer Farbdosieranlage, dort gelagerte Farbdispersionen über Monate hinweg vor mikrobiellen Befall geschützt werden können, da einerseits, wie erwähnt, die Anlagerung und eine folgende Vermehrung von mikrobiellem Befall in der Reinigungsvorrichtung selbst wirksam verhindert wird und damit zusammenhängend auch eine Verbreitung des mikrobiellen Befalls, soweit er bereits besteht, in den mit der Reinigungsvorrichtung zu reinigenden Komponenten verhindert wird. Dies gelingt erfindungsgemäß durch das konsequente Beaufschlagen des oder der mechanischen Reinigungselemente der Reinigungsvorrichtung mit dem Oxidationsmittel. Eine in der Dosieranlage gelagerte Dispersion kann hierbei frei von Konservierungsmitteln sein und weist trotzdem einen langanhaltenden Schutz gegen mikrobiellen Befall auf. Selbst bei häufiger Benutzung der Dosieranlage und einem wiederholten Auffüllen des Behälters mit frischer Dispersion wird bei regelmäßiger Reinigung ein Schutz vor Pilz- und Bakterienbefall durch das erfindungsgemäße Verfahren gewährleistet.

Die Beaufschlagung der wenigstens einen Reinigungsfläche des wenigstens einen Reinigungselements der Reinigungsvorrichtung kann kontinuierlich oder diskontinuierlich, beispielsweise in periodischen Zeitabständen (z.B. alle 6, 12 oder 24 Stunden) erfolgen. Dies bemisst sich u.a. an den Umgebungsbedingungen (Temperatur, Luftfeuchtigkeit, usw.), in denen die Reinigungsvorrichtung zum Einsatz kommt und die eine Entstehung oder Vermehrung mikrobiellen Befalls ggf. begünstigen.

Grundsätzlich eignen sich ganz unterschiedliche Oxidationsmittel zur Verhinderung oder Eliminierung des mikrobiellen Befalls. Es kommen Oxidationsmittel infrage, die gasförmig, flüssig oder fest sind. Flüssige und feste Oxidationsmittel werden bevorzugt als wässrige Lösungen eingesetzt, da hierdurch die Handhabung vereinfacht wird. Erfindungsgemäß ist das Oxidationsmittel ein Oxidationsfluid. Weiter erfindungsgemäß ist das Oxidationsmittel ein gasförmiges Oxidationsfluid.

Um eine möglichst gründliche und vollständige Beaufschlagung der wenigstens einen Reinigungsfläche des wenigstens einen Reinigungselements zu erreichen, ist nach einer vorteilhaften Ausgestaltung der Erfindung vorgesehen, dass das Oxidationsmittel auf die wenigstens eine mechanische Reinigungsfläche aufgesprüht oder aufgeblasen wird. Darüber hinausgehend ist es bevorzugt, dass das Aufsprühen oder Aufblasen des Oxidationsmittels auf die wenigstens eine mechanische Reinigungsfläche im Wesentlichen entlang ihrer Längserstreckung, bevorzugt vollflächig erfolgt. Hierdurch ist sichergestellt, dass es bei der Beaufschlagung keine unbehandelten Flächenabschnitte gibt, an denen der mikrobielle Befall einsetzen kann.

Hinsichtlich der chemischen Zusammensetzung steht eine große Bandbreite von Oxidationsmitteln zur Verfügung. Vorteilhafterweise ist das Oxidationsmittel ein sauerstoff- oder chlorbasiertes Oxidationsmittel oder eine Mischung davon, bevorzugt ein sauerstoff-basiertes Oxidationsmittel. Vorzugsweise wird das Oxidationsmittel ausgewählt aus der Gruppe bestehend aus Natriumhypochlorit, Kaliumhypochlorit, Javelwasser, Chlor, Ozon, Wasserstoffperoxid, Peroxyessigsäure, Perborat, Percarbonat, und Mischungen davon. Diese Oxidationsmittel stellen starke Oxidationsmittel dar, die das Wachstum von mikrobiellen Organismen effektiv unterbinden.

Besonders bevorzugt wird das Oxidationsmittel ausgewählt aus der Gruppe bestehend aus Natriumhypochlorit, Wasserstoffperoxid, Ozon, und Mischungen davon. Die Oxidationsmittel aus der genannten Gruppe haben sich als besonders gut geeignet für den Schutz der Dispersion vor mikrobiellem Befall herausgestellt, da sie effektive Oxidationsmittel darstellen. Ferner sind diese Oxidationsmittel insbesondere bei den zweckmäßigerweise vorgesehenen Mengen für die Dispersion im Wesentlichen unproblematisch. Insbesondere sorgen die genannten Oxidationsmittel bei den zweckmäßigerweise vorgesehenen Mengen nicht zu einer nennenswerten Veränderung der Farbe und/oder Qualität der Dispersion.

Wie erwähnt, ist der Einsatz eines Oxidationsfluids, d.h. eines gasförmigen oder flüssigen Oxidationsmittels, bevorzugt. Erfindungsgemäß wird ein gasförmiges Oxidationsmittel verwendet. Weiter erfindungsgemäß enthält das Oxidationsmittel Ozon. Enthält das Oxidationsmittel Ozon oder wird Ozon als Oxidationsmittel eingesetzt, erfolgt eine besonders langanhaltende antimikrobielle Wirkung. Wird die Reinigungsvorrichtung im Zusammenhang mit einer Dosieranlage für Dispersionen eingesetzt, erweist sich als Vorteil, dass Ozon mit den gängigen Bestandteilen von Dispersionen, insbesondere den gängigen Bestandteilen von Dispersionsfarben oder Pigmentpasten, kompatibel ist. Zudem lässt sich Ozon sehr leicht am Ort der Verwendung herstellen.

Ozon kann beispielsweise aus einer chemischen Reaktion von Kaliumpermanganat mit konzentrierter Schwefelsäure erzeugt werden oder durch Elektrolyse von verdünnter Schwefelsäure, insbesondere bei tiefen Temperaturen. Ozon kann auch aus Luft oder Sauerstoff unter Einwirkung von UV-Strahlung erzeugt werden.

Gemäß einer bevorzugten Ausführungsform des erfindungsgemäßen Verfahrens wird das Ozon in einem Ozongenerator umfassend eine Spannungsquelle, insbesondere einen Hochspannungsgenerator, und eine Entladungseinheit ausgehend von Luft, insbesondere getrockneter Luft, Sauerstoff oder einem Sauerstoffgemisch mit Argon oder Kohlenstoffdioxid erzeugt. Der Aufbau gängiger Ozongeneratoren ist aus dem Stand der Technik bekannt. Gemäß einer gängigen Variante der Ozonherstellung werden in einer Entladungseinheit Sauerstoffmoleküle vorzugsweise durch stille elektrische Entladung, sogenannte *"Koronaentladung",* zu Sauerstoffatomen dissoziiert, wonach noch im Plasma der Entladungsfilamente die Ozonsynthese und Ozonanreicherung stattfindet. Dabei kann der entstehende Ozonanteil an der Endkonzentration des Gasgemisches bei Luft als Ausgangsgas 1 bis 5 Gew.% und bei Sauerstoff als Ausgangsgas 6 bis 13 Gew.% betragen. Vorzugsweise wird Luft als Ausgangsgas zur Ozonerzeugung eingesetzt. Bei der Erzeugung des Ozons in einem Generator wird somit ausgehend von günstigen Startmaterialien auf saubere Weise Ozon hergestellt.

Nach einer besonders vorteilhaften Ausgestaltung der Erfindung wird die wenigstens eine Reinigungsfläche des wenigstens einen mechanischen Reinigungselements im Betrieb der Reinigungsvorrichtung zumindest zeitweise in einem Reinigungsbad gereinigt, wobei das Oxidationsmittel optional auch das Reinigungsbad zumindest zeitweise beaufschlagt.

Bei dieser Ausgestaltung der Reinigungsvorrichtung wird die wenigstens eine Reinigungsfläche des wenigstens einen mechanischen Reinigungselements selbst gereinigt, so dass die an der wenigstens einen Reinigungsfläche anhaftenden mikrobiellen Verunreinigungen von der Reinigungsfläche abgewaschen werden. Hierdurch kann die Reinigungsvorrichtung über einen längeren Zeitraum völlig autark operieren. Da der mikrobielle Befall somit von der Reinigungsfläche in das Reinigungsbad gelangt, kann es sinnvoll sein, dass neben der Reinigungsfläche auch das Reinigungsbad selbst mit dem Oxidationsmittel zumindest zeitweise beaufschlagt wird. In diesem Fall kann also der mikrobielle Befall noch wirksamer bekämpft werden, da Rückstände mikrobiellen Befalls im Reinigungsbad direkt bekämpft werden.

Nach einer weiteren vorteilhaften Ausgestaltung der Erfindung erfolgt das Aufsprühen oder Aufblasen des Oxidationsmittels auf die wenigstens eine Reinigungsfläche des wenigstens einen mechanischen Reinigungselements außerhalb des Reinigungsbads. Hierdurch kann das Oxidationsmittel noch gezielter auf die wenigstens eine Reinigungsfläche geleitet werden.

Das mechanische Reinigungselement der Reinigungsvorrichtung weist erfindungsgemäß wenigstens eine Reinigungsfläche auf. Diese kann unterschiedlich gestaltet sein. Bevorzugt wird eine umlaufende Reinigungsfläche, insbesondere bei zylindrischer Ausgestaltung des mechanischen Reinigungselements. Es versteht sich, dass anstelle einer zylindrischen Auslegung mit kreisförmiger Grundfläche auch elliptische oder mehreckige Grundflächen möglich sind. Anstelle einer umlaufenden Reinigungsfläche sind ferner auch mehrere nicht miteinander zusammenhängende Reinigungsflächen möglich, beispielsweise eine Mehrzahl im Wesentlichen ebener Reinigungsflächen, welche beispielsweise radial nach außen weisend, um eine gemeinsame Achse drehend angeordnet sein können.

Bei der Ausgestaltung der wenigstens einen Reinigungsfläche ist zu berücksichtigen, dass grundsätzlich sämtliche mechanische Reinigungstechniken im Rahmen der vorliegenden Erfindung anwendbar sind, wie beispielsweise Wischen, Schrubben, Schleifen usw.. Orientiert an der jeweils gewählten Reinigungstechnik ist die Reinigungsfläche entsprechend gestaltet, beispielsweise als Bürste oder Schwamm. Nach einer vorteilhaften Ausgestaltung der Erfindung ist das wenigstens eine Reinigungselement als Bürste und seine Reinigungsfläche entsprechend als Bürstenfläche ausgestaltet. Besonders bevorzugt ist dabei die Ausgestaltung als zylindrische oder walzenförmige Bürste. Unabhängig von der geometrischen Gestaltung der Bürstenfläche wird das Oxidationsmittel bevorzugt im Wesentlichen parallel zu den Borsten in die Bürste eingesprüht oder eingeblasen, so dass die Borsten entlang ihrer gesamten Länge von mikrobiellem Befall gereinigt werden können, was zu einem besonders gründlichen Reinigungsergebnis führt.

Ist das wenigstens eine mechanische Reinigungselement als zylindrische oder walzenförmig rotierende Bürste ausgelegt und kommt ferner ein Reinigungsbad für das Reinigungselement zum Einsatz, so kann die Bürste im Betrieb der Reinigungsvorrichtung teilweise in das Bad eingetaucht sein mit der Folge, dass die Bürste während der Reinigung selbst ständig im Reinigungsbad gereinigt bzw. gespült wird, was einen hohen Automatisierungsgrad ermöglicht.

In Bezug auf die konstruktive Ausgestaltung der Zuführung des Oxidationsmittels besteht eine bevorzugte Lösung darin, dass das Oxidationsmittel über wenigstens eine Zuführleitung zugeführt wird, wobei die wenigstens eine Zuführleitung im Wesentlichen entlang der Längserstreckung der wenigstens einen Reinigungsfläche des wenigstens einen mechanischen Reinigungselements angeordnet ist. Dabei weist die wenigstens eine Zuführleitung ferner eine, bevorzugt eine Mehrzahl, von auf die wenigstens eine Reinigungsfläche ausgerichtete Auslassöffnungen zum Aufsprühen oder Aufblasen des Oxidationsmittels auf. Diese konstruktive Lösung zeichnet sich durch einen einfachen und schnell zu realisierenden Aufbau aus und ermöglicht, dass die wenigstens eine Reinigungsfläche des wenigstens einen mechanischen Reinigungselements möglichst vollflächig mit Oxidationsmittel beaufschlagt wird. Insbesondere eignet sich diese konstruktive Lösung für den Einsatz zylindrischer oder walzenförmiger mechanischer Reinigungselemente, wie zylindrischer oder walzenförmiger Schwämme oder Bürsten.

Ein weiterer Aspekt der vorliegenden Erfindung betrifft ein Verfahren zum Reinigen einer Dosieranlage, insbesondere für eine Dispersion, speziell einer Farbdosieranlage, wobei die Dosieranlage wenigstens eine Dosiereinheit mit einem Pumpenkopf aufweist, wobei der Pumpenkopf mittels einer Reinigungsvorrichtung mit wenigstens einem wenigstens eine Reinigungsfläche aufweisenden mechanischen Reinigungselement gereinigt wird, wobei ein mikrobieller Befall der Reinigungsvorrichtung mit einem Verfahren nach einem der Ansprüche 1 bis 11 vermieden wird.

Für die Vorteile dieses Reinigungsverfahrens gelten die vorstehend erläuterten Vorteile entsprechend. Insbesondere kommen die Vorteile beim Einsatz des Reinigungsverfahrens bei einer Farbdosieranlage mit einer Mehrzahl von Dosiereinheiten zu Tragen, da hier durch das erfindungsgemäße Verfahren die Ausbreitung eines mikrobiellen Befalls von einer Dosiereinheit auf mehrere oder alle anderen Dosiereinheiten wirksam vermieden werden kann, was sich in den aus der Praxis bekannten Reinigungsverfahren stets als besonderes Problem darstellte.

Vorrichtungsmäßig wird die eingangs gestellte Aufgabe mit einer Reinigungsvorrichtung für eine Dosieranlage, insbesondere für eine Dispersion, speziell für eine Farbdosieranlage, umfassend wenigstens ein mechanisches Reinigungselement mit wenigstens einer Reinigungsfläche sowie eine Zuführeinheit für ein Oxidationsmittel zur zumindest zeitweisen Beaufschlagung der wenigstens einen Reinigungsfläche mit einem Oxidationsmittel gelöst, wobei die Reinigungsvorrichtung zum Reinigen der Dosieranlage gemäß Anspruch 12 konfiguriert ist.

Wiederum gilt für die Vorteile der erfindungsgemäßen Vorrichtung das vorstehend Gesagte entsprechend.

Nach einer vorteilhaften Ausgestaltung der Reinigungsvorrichtung umfasst die Reinigungsvorrichtung ein Reinigungsbad zum zumindest zeitweisen Reinigen der wenigstens einen Reinigungsfläche des wenigstens einen mechanischen Reinigungselements im Betrieb der Reinigungsvorrichtung.

Ein weiterer Aspekt der vorliegenden Erfindung betrifft eine Dosieranlage für eine Dispersion, insbesondere Farbdosieranlage, umfassend eine Reinigungsvorrichtung nach Anspruch 13 oder 14.

Die erfindungsgemäße Dosieranlage kann neben der erfindungsgemäßen, mit Oxidationsmittel beaufschlagbaren Reinigungsvorrichtung auch noch Vorrichtungen zur Vermeidung des mikrobiellen Befalls anderer Teile der Dosieranlage, insbesondere der in dem/den Behälter(n) befindlichen Dispersion(en), umfassen. Insbesondere kann die Dosieranlage eine Vorrichtung umfassen, mit welcher ein Oxidationsmittel wie z.B. Ozon in einen oder mehrere der Behälter, in denen die Dispersion gelagert ist, eingebracht wird, wie in der internationalen Patentanmeldung PCT/EP2019/052537 beschrieben (vgl. insbesondere Fig. 1 und die dazugehörige Figurenbeschreibung der PCT/EP2019/052537). Das Einbringen von Oxidationsmittel, insbesondere von Ozon, in den/die Behälter der Dosieranlage kann beispielsweise mit Hilfe von geeigneten Adaptern erfolgen, wie in der internationalen Patentanmeldung PCT/EP2019/052537 beschrieben (vgl. insbesondere Fig. 3, 5, 6, 7a, 7b, 8a und 8b sowie die dazugehörigen Figurenbeschreibungen der PCT/EP2019/052537). Auf Offenbarung in der internationalen Patentanmeldung PCT/EP2019/052537 wird in diesem Zusammenhang ausdrücklich Bezug genommen. Ein zumindest zeitweises Beaufschlagen der Reinigungsfläche des wenigstens einen mechanischen Reinigungselements der Reinigungsvorrichtung, wie hierin beschrieben, kombiniert mit einem Einbringen von Oxidationsmittel in einen oder mehrere der Behälter, in denen die Dispersion gelagert ist, wie in der PCT/EP2019/052537 beschrieben, stellt eine besonders bevorzugte Ausführungsform der vorliegenden Erfindung dar.

Im Folgenden wir die vorliegende Erfindung anhand einer ein Ausführungsbeispiel der Erfindung darstellenden Zeichnung erläutert. Es zeigen:
- Fig. 1A: eine Dosieranlage für eine Dispersionsfarbe mit einer Reinigungsvorrichtung in schematisierter Draufsicht,
- Fig. 1B: die Dosieranlage der Fig. 1A in schematisierter Seitenansicht,
- Fig. 2: die Reinigungsvorrichtung der Dosieranlage der Fig. 1 in perspektivischer Ansicht,
- Fig. 3: die Reinigungsvorrichtung der Fig. 2 im Querschnitt gemäß Schnittlinie III-III aus Fig. 2,
- Fig. 4A: die Zuführung eines Oxidationsmittels der Reinigungsvorrichtung der Fig. 2 in vergrößerter perspektivischer Ansicht und
- Fig. 4B: die Zuführung eines Oxidationsmittels der Reinigungsvorrichtung der Fig. 2 in zu Fig. 4A gedrehter perspektivischer Ansicht.

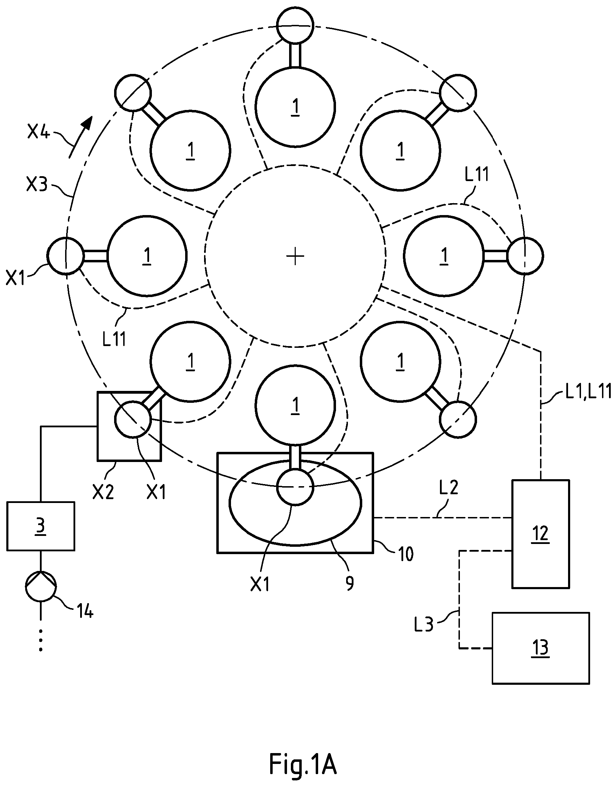

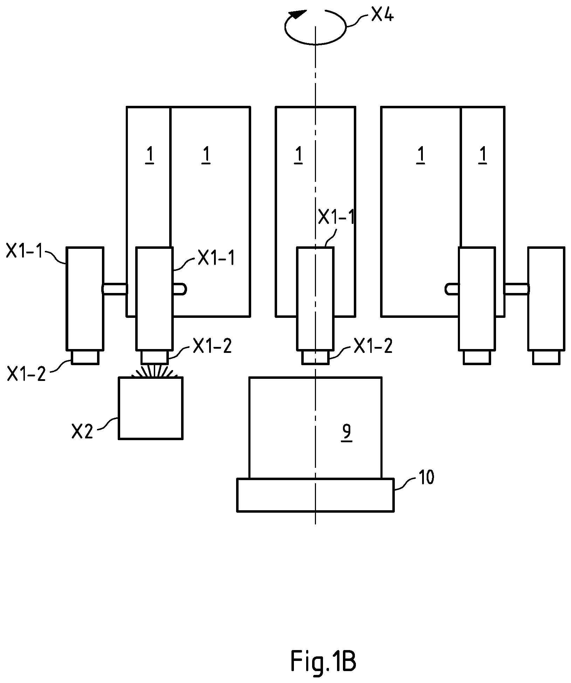

Fig. 1 zeigt eine bevorzugte Ausführungsform einer Dosieranlage zum Mischen einer Dispersionsfarbe mit einer Reinigungsvorrichtung in schematisierter Draufsicht. Es versteht sich, dass prinzipiell der gleiche Aufbau auch zur Dosierung und/oder Mischung anderer Materialien genutzt werden kann.

Die Dosieranlage der Fig. 1 umfasst im Einzelnen eine Mehrzahl vorliegend karussellartig auf einem mit strichpunktierter Linie dargestellten Teller X4 angeordneter Behälter 1, wobei jeder Behälter 1 über eine Zuleitung mit einer Dosiereinheit X1 verbunden ist. Über eine Steuerleitung L1 ist der Antrieb des Tellers X4 mit einem Steuerungsrechner 12 verbunden. Ferner sind die Dosiereinheiten X1 jeweils über Steuerleitungen L11 mit dem Steuerungsrechner verbunden. Wie in der Draufsicht der Fig. 1 erkennbar, ist die Dosiereinheit X1 eines der Farbbehälter 1 über einem Aufnahmebehältnis, vorliegend einem Eimer 9, angeordnet, so dass die in diesem Behälter 1 gelagerte Dispersion über die zugehörige Dosiereinheit X1 in den Eimer 9 dosiert werden kann. Der Eimer 9 seinerseits steht auf einer Waage 10, die über eine Steuerleitung L2 mit dem Steuerungsrechner 12 verbunden ist. Der Steuerungsrechner 12 wiederum ist über eine Steuerleitung L3 mit einem Etikettendrucker 13 verbunden.

Die bezogen auf die über dem Eimer 9 positionierte Dosiereinheit X1 im Uhrzeigersinn X5 benachbarte Dosiereinheit X1 ist oberhalb einer Reinigungsvorrichtung X2 derart positioniert, dass die in den Fig. 1B bis 4B noch im Detail beschriebene Reinigungsvorrichtung X2 den Pumpenkopf X6 (vgl. Fig. 1B) der Dosiereinheit X1 reinigen kann. Wie im Folgenden noch näher erläutert wird, umfasst die Reinigungsvorrichtung X2 ein mechanisches Reinigungselement in Form einer rotierenden zylindrischen Bürste X29 mit einer als Bürstenfläche ausgebildeten zylindrischen Reinigungsfläche X31, mithilfe derer der Pumpenkopf X6 einer jeden Dosiereinheit X1 von Farbrückständen gereinigt werden kann.

Wie in Fig. 1A ebenfalls schematisch dargestellt, ist die Reinigungsvorrichtung X2 über eine Zuführleitung mit einem Ozongenerator 3 verbunden, in den wiederum über eine Membranpumpe 14 Luft oder Sauerstoff eingeleitet wird. Im Ozongenerator 3, wird beispielsweise mittels einer Koronaentladung Ozon erzeugt, welches über die Zuführleitung X24 (in Fig. 1A nur schematisch dargestellt) in die Reinigungsvorrichtung X2 zwecks Beaufschlagung der rotierenden Bürste X29 eingeleitet wird.

Im Betrieb der Dosieranlage berechnet der Steuerungsrechner 12 nach kundenseitiger Eingabe eines gewünschten Farbtons und des gewünschten Volumens die Anteile der in den Behältern 1 gespeicherten Grundfarben und steuert den Antrieb des Tellers X4 derart an, dass nacheinander die Dosiereinheiten X1 der Behälter 1 mit den erforderlichen Grundfarben über dem Eimer 9 positioniert werden und die berechnete Menge in den Eimer 9 abgegeben wird, um so den gewünschten Farbton im Eimer 9 zu erhalten. Dies wird mittels der mit dem Steuerungsrechner über die Leitung L2 verbundenen Waage 10 überwacht. Der Antrieb des Tellers X4 wird derart angesteuert, dass er sich stets im UhrzeigersinnX5 dreht. Dies führt dazu, dass der Pumpenkopf X6 einer jeden am Farbmischprozess beteiligten Dosiereinheit X1 unmittelbar nach Einsatz durch die Reinigungsvorrichtung X2 gereinigt und somit von Dispersionsrückständen befreit wird. Über den Etikettendrucker 13 wird das passende Etikett für die Farbmischung gedruckt.

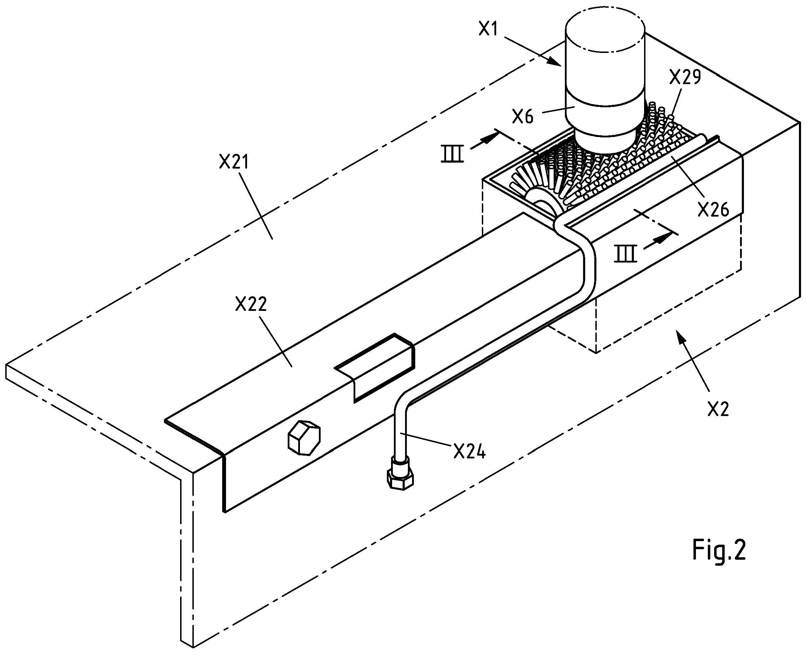

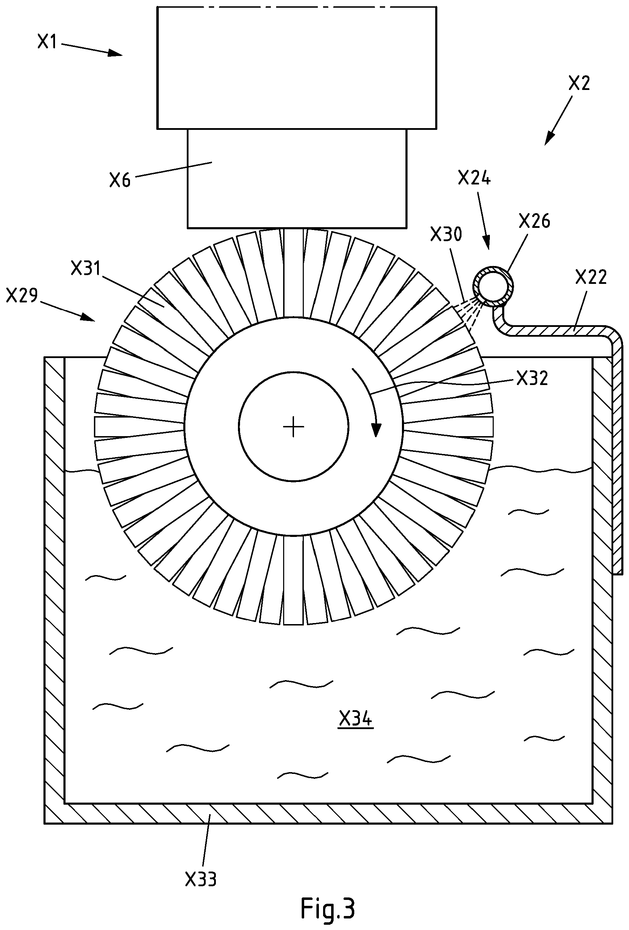

Fig. 2 und 3 zeigen die Reinigungsvorrichtung X2 der Dosieranlage der Fig. 1 in perspektivischer Ansicht sowie im Querschnitt gemäß Schnittlinie III-III aus Fig. 2.

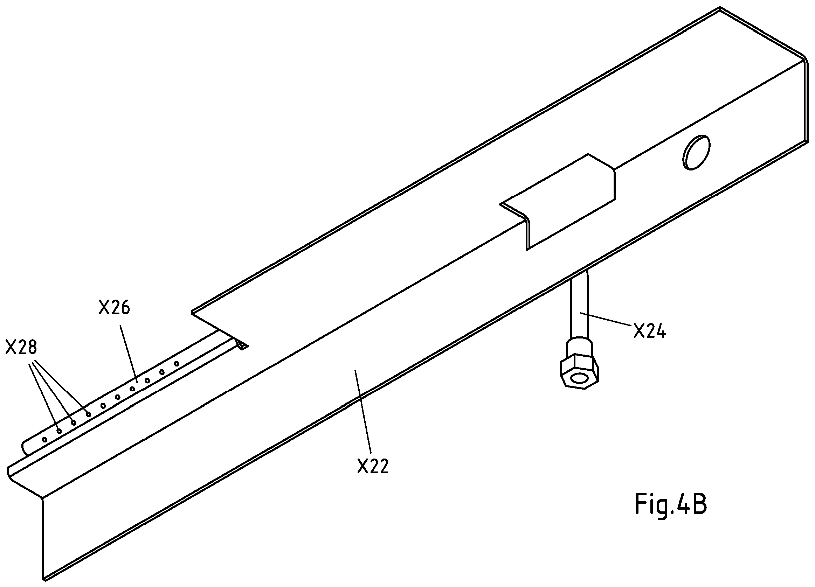

Gemäß Fig. 3 umfasst die Reinigungsvorrichtung X2 als zentrales Element eine in der vorliegenden Ansicht im Uhrzeigersinn X32 rotierende Bürste X31, die im Betrieb mit den oberseitig angeordneten Borsten der Bürstenfläche X31 den Pumpenkopf X6 von Dispersionsrückständen reinigt, während die unterseitigen in ein Reinigungsbad X34 eingetauchten Borsten der Bürste X29 in dem Reinigungsbad X34 von den Farbrückständen befreit werden. Das Reinigungsbad X34 wird vorliegend durch ein Wasserbad gebildet, welches sich in einem Behälter X33 befindet. Wie insbesondere in Fig. 2 erkennbar ist, wird über die Zuführleitung X24 ein Ozon enthaltender Gasstrom als Oxidationsmittel über eine Mehrzahl von in einem Austrittsbereich X26 der Zuführleitung angeordnete Austrittsöffnungen X28 (vgl. Fig. 4B) auf die Bürste X29 geblasen, wobei der Gasstrom im Wesentlichen parallel zu den Borsten der Bürstenfläche X31, d.h. im Wesentlichen radial in die Bürste eingeblasen wird. Der Behälter X33 des Reinigungsbades X34 ist vorliegend über ein Befestigungsprofil X22 mit einem Gehäuse X21 verbunden.

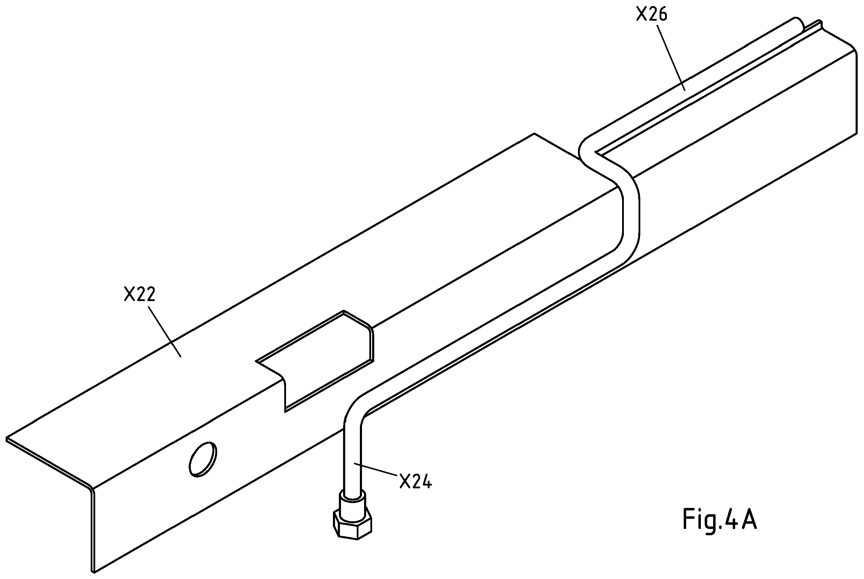

Die Befestigungsschiene X22 samt Zuführleitung X24 für den Ozon enthaltenden Gasstrom ist in den Fig. 4A und 4B nochmals in zweifacher Ansicht perspektivisch dargestellt, wobei in der Darstellung der Fig. 4B die Austrittsöffnungen X28 für den Gasstrom im parallel zur Achse der Bürste X29 ausgerichteten Austrittsbereich X26 der Zuführleitung X24 deutlich erkennbar sind.

Der besondere Vorteil der Reinigungsvorrichtung sowie des Reinigungsverfahrens besteht darin, dass weder im Reinigungsbad X34 noch in der Bürste X29 die Gefahr des mikrobiellen Befalls mit Bakterien und Pilzen zu befürchten ist, da dies durch das eingeblasene Ozon als Oxidationsmittel wirksam verhindert wird. Demnach erweist sich das erfindungsgemäß Verfahren zur Vermeidung des mikrobiellen Befalls der Reinigungsvorrichtung X2 als äußerst wirksam. Zudem ist die Gefahr einer Kontamination anderer Pumpenköpfe X6 durch die Reinigungsvorrichtung X2 minimiert, auch wenn diese eine mikrobiell befallenen Pumpenkopf X6 reinigt - und sich damit entsprechende Verunreinigungen auf der zylindrischen Bürstenfläche X31 und im Reinigungsbad X34 sammeln - und im Anschluss daran weitere Pumpenköpfe X6 gereinigt werden.

## Patentansprüche

1. Verfahren zur Vermeidung des mikrobiellen Befalls einer Reinigungsvorrichtung (X2) für eine Dosieranlage, wobei die Reinigungsvorrichtung (X2) wenigstens ein mechanisches Reinigungselement (X29) mit wenigstens einer Reinigungsfläche (X31) aufweist,
**dadurch gekennzeichnet, dass**
die wenigstens eine Reinigungsfläche (X31) des wenigstens einen mechanischen Reinigungselements (X29) zumindest zeitweise mit einem Oxidationsmittel beaufschlagt wird, wobei das Oxidationsmittel ein gasförmiges Oxidationsfluid ist und wobei das Oxidationsfluid Ozon enthält.

2. Verfahren nach Anspruch 1,
**dadurch gekennzeichnet, dass**
das Oxidationsmittel auf die wenigstens eine mechanische Reinigungsfläche (X31) aufgeblasen wird.

3. Verfahren nach Anspruch 2,
**dadurch gekennzeichnet, dass**
das Aufblasen des Oxidationsmittels auf die wenigstens eine Reinigungsfläche (X31) im Wesentlichen entlang ihrer Längserstreckung, bevorzugt vollflächig erfolgt.

4. Verfahren nach einem der Ansprüche 1 bis 3,
**dadurch gekennzeichnet, dass**
die wenigstens eine Reinigungsfläche (X31) des wenigstens einen mechanischen Reinigungselements (X29) im Betrieb der Reinigungsvorrichtung (X2) zumindest zeitweise in einem Reinigungsbad (X34) gereinigt wird, wobei optional auch das Reinigungsbad (X34) mit dem Oxidationsmittel zumindest zeitweise beaufschlagt wird.

5. Verfahren nach Anspruch 4,
**dadurch gekennzeichnet, dass**
das Oxidationsmittel auf die wenigstens eine mechanische Reinigungsfläche (X31) aufgeblasen wird und das Aufblasen des Oxidationsmittels auf die wenigstens eine Reinigungsfläche (X31) des wenigstens einen mechanischen Reinigungselements (X29) außerhalb des Reinigungsbads (X34) erfolgt.

6. Verfahren nach einem der Ansprüche 1 bis 5,
**dadurch gekennzeichnet, dass**
das wenigstens eine mechanische Reinigungselement (X2) eine umlaufende Reinigungsfläche (X31) aufweist.

7. Verfahren nach einem der Ansprüche 1 bis 6,
**dadurch gekennzeichnet, dass**
das wenigstens eine mechanische Reinigungselement (X29) zylindrisch oder walzenförmig ausgebildet ist.

8. Verfahren nach einem der Ansprüche 1 bis 7,
**dadurch gekennzeichnet, dass**
das wenigstens eine mechanische Reinigungselement (X29) wenigstens eine Bürste umfasst.

9. Verfahren nach Anspruch 8,
**dadurch gekennzeichnet, dass**
das wenigstens eine mechanische Reinigungselement (X29) als rotierende zylindrische Bürste ausgebildet ist.

10. Verfahren nach Anspruch 8 oder 9,
**dadurch gekennzeichnet, dass**
das Oxidationsmittel im Wesentlichen parallel zu den Borsten in die Bürste eingeblasen wird.

11. Verfahren nach einem der Ansprüche 1 bis 10,
**dadurch gekennzeichnet, dass**
das Oxidationsmittel über wenigstens eine Zuführleitung (X24, X26) zugeführt wird, wobei die wenigstens eine Zuführleitung (X24, X26) im Wesentlichen entlang der Längserstreckung der wenigstens einen Reinigungsfläche (X31) des wenigstens einen mechanischen Reinigungselements (X29) angeordnet ist, wobei die wenigstens eine Zuführleitung (X24, X26) eine, bevorzugt eine Mehrzahl, von auf die wenigstens eine Reinigungsfläche (X31) ausgerichtete Auslassöffnungen (X28) zum Aufblasen des Oxidationsmittels aufweist,

12. Verfahren zum Reinigen einer Dosieranlage, insbesondere für eine Dispersion, speziell einer Farbdosieranlage, wobei die Dosieranlage wenigstens eine Dosiereinheit (X1) mit einem Pumpenkopf (X6) aufweist, wobei der Pumpenkopf (X6) mittels einer Reinigungsvorrichtung (X2) mit wenigstens einem wenigstens eine Reinigungsfläche (X31) aufweisenden mechanischen Reinigungselement (X29) gereinigt wird,
**dadurch gekennzeichnet, dass**
ein mikrobieller Befall der Reinigungsvorrichtung (X2) mit einem Verfahren nach einem der Ansprüche 1 bis 11 vermieden wird.

13. Reinigungsvorrichtung (X2) für eine Dosieranlage, insbesondere für eine Dispersion, speziell für eine Farbdosieranlage, umfassend wenigstens ein mechanisches Reinigungselement (X29) mit wenigstens einer Reinigungsfläche (X31) sowie eine Zuführeinheit (X24, X26) für ein Oxidationsmittel zur zumindest zeitweisen Beaufschlagung der wenigstens einen Reinigungsfläche (X31) mit einem Oxidationsmittel, wobei die Reinigungsvorrichtung (X2) konfiguriert ist zum Reinigen der Dosieranlage gemäß Anspruch 12.

14. Reinigungsvorrichtung nach Anspruch 13,
**dadurch gekennzeichnet, dass**
die Reinigungsvorrichtung (X2) ein Reinigungsbad (X34) zum zumindest zeitweisen Reinigen der wenigstens einen Reinigungsfläche (X31) des wenigstens einen mechanischen Reinigungselements (X29) im Betrieb der Reinigungsvorrichtung (X2) umfasst.

15. Dosieranlage für eine Dispersion, insbesondere Farbdosieranlage, umfassend eine Reinigungsvorrichtung (X2) nach Anspruch 13 oder 14.

## Claims

1. Method for preventing the microbial attack of a cleaning device (X2) for a dosing system, the cleaning device (X2) comprising at least one mechanical cleaning element (X29) which has at least one cleaning surface (X31),
**characterized in that**
the at least one cleaning surface (X31) of the at least one mechanical cleaning element (X29) is at least temporarily exposed to an oxidizing agent, the oxidizing agent being a gaseous oxidizing fluid and the oxidizing fluid containing ozone.

2. Method according to claim 1,
**characterized in that**
the oxidizing agent is blown onto the at least one mechanical cleaning surface (X31).

3. Method according to claim 2,
**characterized in that**
the oxidizing agent is blown onto the at least one cleaning surface (X31) substantially along its longitudinal extent, preferably over the entire surface thereof.

4. Method according to any of claims 1 to 3,
**characterized in that**
the at least one cleaning surface (X31) of the at least one mechanical cleaning element (X29) is cleaned at least temporarily in a cleaning bath (X34) during operation of the cleaning device (X2), optionally the cleaning bath (X34) also being at least temporarily exposed to the oxidizing agent.

5. Method according to claim 4,
**characterized in that**
the oxidizing agent is blown onto the at least one mechanical cleaning surface (X31) and the blowing of the oxidizing agent onto the at least one cleaning surface (X31) of the at least one mechanical cleaning element (X29) takes place outside the cleaning bath (X34).

6. Method according to any of claims 1 to 5,
**characterized in that**
the at least one mechanical cleaning element (X2) has a circumferential cleaning surface (X31).

7. Method according to any of claims 1 to 6,
**characterized in that**
the at least one mechanical cleaning element (X29) is cylindrical or roller-shaped.

8. Method according to any of claims 1 to 7,
**characterized in that**
the at least one mechanical cleaning element (X29) comprises at least one brush.

9. Method according to claim 8,
**characterized in that**
the at least one mechanical cleaning element (X29) is designed as a rotating cylindrical brush.

10. Method according to claim 8 or 9,
**characterized in that**
the oxidizing agent is blown into the brush substantially in parallel with the bristles.

11. Method according to any of claims 1 to 10,
**characterized in that**
the oxidizing agent is supplied via at least one supply line (X24, X26), the at least one supply line (X24, X26) being arranged substantially along the longitudinal extent of the at least one cleaning surface (X31) of the at least one mechanical cleaning element (X29), the at least one supply line (X24, X26) having one, preferably a plurality of outlet openings (X28) aligned with the at least one cleaning surface (X31) for blowing the oxidizing agent.

12. Method for cleaning a dosing system, in particular for a dispersion, especially a paint dosing system, the dosing system comprising at least one dosing unit (X1) having a pump head (X6), the pump head (X6) being cleaned by means of a cleaning device (X2) which comprises at least one mechanical cleaning element (X29) having at least one cleaning surface (X31),
**characterized in that**
a microbial attack of the cleaning device (X2) is prevented by a method according to any of claims 1 to 11.

13. Cleaning device (X2) for a dosing system, in particular for a dispersion, especially for a paint dosing system, comprising at least one mechanical cleaning element (X29) having at least one cleaning surface (X31) and comprising a supply unit (X24, X26) for an oxidizing agent for at least temporarily applying an oxidizing agent to the at least one cleaning surface (X31), wherein the cleaning device (X2) is configured to clean the dosing system according to claim 12.

14. Cleaning device according to claim 13,
**characterized in that**
the cleaning device (X2) comprises a cleaning bath (X34) for at least temporarily cleaning the at least one cleaning surface (X31) of the at least one mechanical cleaning element (X29) during operation of the cleaning device (X2).

15. Dosing system for a dispersion, in particular a paint dosing system, comprising a cleaning device (X2) according to claim 13 or 14.

## Revendications

1. Procédé permettant d'éviter l'attaque microbienne d'un dispositif de nettoyage (X2) pour une installation de dosage, dans lequel le dispositif de nettoyage (X2) présente au moins un élément de nettoyage mécanique (X29) comportant au moins une surface de nettoyage (X31),
**caractérisé en ce que**
l'au moins une surface de nettoyage (X31) de l'au moins un élément de nettoyage mécanique (X29) est exposée au moins temporairement à un agent oxydant, dans lequel l'agent oxydant est un fluide oxydant gazeux et dans lequel le fluide oxydant contient de l'ozone.

2. Procédé selon la revendication 1,
**caractérisé en ce que**
l'agent oxydant est soufflé sur l'au moins une surface de nettoyage (X31) mécanique.

3. Procédé selon la revendication 2,
**caractérisé en ce que**
le soufflage de l'agent oxydant sur l'au moins une surface de nettoyage (X31) est effectué sensiblement le long de son extension longitudinale,
de préférence sur toute la surface.

4. Procédé selon l'une des revendications 1 à 3,
**caractérisé en ce que**
l'au moins une surface de nettoyage (X31) de l'au moins un élément de nettoyage mécanique (X29) est nettoyée au moins temporairement dans un bain de nettoyage (X34) pendant le fonctionnement du dispositif de nettoyage (X2), dans lequel le bain de nettoyage (X34) est éventuellement également exposé au moins temporairement à l'agent oxydant.

5. Procédé selon la revendication 4,
**caractérisé en ce que**
l'agent oxydant est soufflé sur l'au moins une surface de nettoyage (X31) mécanique et le soufflage
de l'agent oxydant sur l'au moins une surface de nettoyage (X31) de l'au moins un élément de nettoyage mécanique (X29) est effectué hors du bain de nettoyage (X34).

6. Procédé selon l'une des revendications 1 à 5,
**caractérisé en ce que**
l'au moins un élément de nettoyage mécanique (X2) présente une surface de nettoyage (X31) circonférentielle.

7. Procédé selon l'une des revendications 1 à 6,
**caractérisé en ce que**
l'au moins un élément de nettoyage mécanique (X29) est réalisé cylindrique ou en forme de rouleau.

8. Procédé selon l'une des revendications 1 à 7,
**caractérisé en ce que**
l'au moins un élément de nettoyage mécanique (X29) comprend au moins une brosse.

9. Procédé selon la revendication 8,
**caractérisé en ce que**
l'au moins un élément de nettoyage mécanique (X29) est réalisé sous forme de brosse cylindrique rotative.

10. Procédé selon la revendication 8 ou 9,
**caractérisé en ce que**
l'agent oxydant est insufflé dans la brosse sensiblement parallèlement aux poils.

11. Procédé selon l'une des revendications 1 à 10,
**caractérisé en ce que**
l'agent oxydant est amené par l'intermédiaire d'au moins une conduite d'amenée (X24, X26), dans lequel l'au moins une conduite d'amenée (X24, X26) est disposée sensiblement le long de l'extension longitudinale de l'au moins une surface de nettoyage (X31) de l'au moins un élément de nettoyage mécanique (X29), dans lequel l'au moins une conduite d'amenée (X24, X26) présente une, de préférence une pluralité d'ouvertures de sortie (X28) orientées vers l'au moins une surface de nettoyage (X31) pour le soufflage de l'agent oxydant.

12. Procédé permettant le nettoyage d'une installation de dosage, en particulier pour une dispersion, spécialement une installation de dosage de peinture, dans lequel l'installation de dosage présente au moins une unité de dosage (X1) comportant une tête de pompe (X6), dans lequel la tête de pompe (X6) est nettoyée au moyen d'un dispositif de nettoyage (X2) comportant au moins un élément de nettoyage mécanique (X29) présentant au moins une surface de nettoyage (X31),
**caractérisé en ce que**
une attaque microbienne du dispositif de nettoyage (X2) est évitée par un procédé selon l'une des revendications 1 à 11.

13. Dispositif de nettoyage (X2) pour une installation de dosage, en particulier pour une dispersion, spécialement pour une installation de dosage de peinture, comprenant au moins un élément de nettoyage mécanique (X29) comportant au moins une surface de nettoyage (X31) ainsi qu'une unité d'amenée (X24, X26) pour un agent oxydant pour l'exposition au moins temporaire de l'au moins une surface de nettoyage (X31) à un agent oxydant, dans lequel le dispositif de nettoyage (X2) est configuré pour nettoyer l'installation de dosage selon la revendication 12.

14. Dispositif de nettoyage selon la revendication 13,
**caractérisé en ce que**
le dispositif de nettoyage (X2) comprend un bain de nettoyage (X34) pour le nettoyage au moins temporaire de l'au moins une surface de nettoyage (X31) de l'au moins un élément de nettoyage mécanique (X29) pendant le fonctionnement du dispositif de nettoyage (X2).

15. Installation de dosage pour une dispersion, en particulier installation de dosage de peinture, comprenant un dispositif de nettoyage (X2) selon la revendication 13 ou 14.
